# EUROPEAN PATENT APPLICATION

(11) **EP 2 957 631 A1**
(43) Date of publication of application: **23.12.2015**
(21) Application number: 14751813.8
(22) Date of filing: 07.02.2014
(51) Int. Cl.: C12N 5/073, A61K 6/02, A61L 27/00

(54) **METHOD FOR PRODUCING A PLURALITY OF TEETH FROM ONE ISOLATED TOOTH GERM**

(30) Priority: 13.02.2013 JP 2013025978
(71) Applicant: ORGAN TECHNOLOGIES, INC., Tokyo 1080074 (JP)
(72) Inventor: TSUJI, Takashi, Tokyo 162-8601 (JP); OSHIMA, Masamitsu, Tokyo 162-8601 (JP)
(74) Representative: Heubeck, Christian
(86) International application number: PCT/JP2014/052858
(87) International publication number: WO 2014/126007

(57) **Abstract**

The object of the present invention is to provide a method for manufacturing multiple teeth from one isolated tooth germ in order to increase the absolute number of tooth germs that can be employed for transplantation.

The present invention relates to a method for manufacturing a tooth. More specifically, the present invention provides a method for manufacturing multiple teeth from one isolated tooth germ comprising the epithelial and mesenchymal tissue layers characterized in that it comprises the steps of: (a) completely or partially splitting one isolated tooth germ comprising the epithelial and mesenchymal tissue layers, wherein said splitting is characterized in splitting so that each splitted tooth germ portion respectively comprises a portion of said epithelial tissue layer and a portion of said mesenchymal tissue layer, and (b) culturing said splitted tooth germs in vitro or culturing in vivo in a living animal other than humans to form multiple teeth.

## Description

### Technical Field

The present invention relates to a method for manufacturing multiple teeth from one isolated tooth germ comprising the epithelial and mesenchymal tissue layers.

### Background Art

There are expectations for "organ regenerative medicine," which refers to replacement of a regenerated organ which is multiple types of cells that have been three-dimensionally reconstructed for an organ that has dysfunctioned. In the dental field, although therapeutic substitution by an artificial material has been applied for tooth loss and thus establishing an effective medical care technology for restoring the mastication function, there are expectations to expand this to "dental regenerative therapy" that is more functional and improves QOL to a higher level.

A cell manipulation technology for fabricating a tooth germ that is capable of development similarly to a normal tooth germ from single cell state tooth germ-derived cells has recently been developed (Non-Patent Literature 1). It became clear that this tooth germ develops normally in the oral cavity, and is also functionally equivalent to a natural tooth (Non-Patent Literature 2). However, since the number of tooth germs a patient possesses is limited, "securing the number of tooth germs" is an issue for realization of "tooth regeneration" that employs tooth germs.

Moreover, tooth germ transplantation therapy, which is transplanting an autologous tooth germ in the juvenile developmental stage to a patient who has developed congenital and acquired tooth loss, has been clinically performed. This therapeutic method not only allows regeneration of functional tooth by biological tooth germ development, but is thought to be a realistically useful dental regenerative therapy in terms of practical application of tooth regenerative therapy in humans. However, since transplantatable tooth germs are restricted by the number or the developmental stage of tooth germs, "securing the number of tooth germs" as transplantation material is again an issue.

### Citation List

[Non-Patent Literature 1] Nature Methods 4:227-230, 2007
[Non-Patent Literature 2] PNAS 11; 106, 13475-80, 2009

### Summary of the Invention

### Problem to be Solved by the Invention

The object of the present invention is to provide a method for manufacturing multiple teeth from one isolated tooth germ in order to increase the absolute number of tooth germs that can be employed for transplantation.

### Means for Solving the Problems

As a result of repeated investigations by the present inventors to solve the above problem, focus was set on the fact that regulative eggs represented by those of frogs, newt, and sea urchins develop multiple complete individuals by ligation with a ligature or by cleaving, and it was found that multiple teeth germs may be manufactured from one tooth germ by applying this method to tooth germs to complete the present invention.

In other words, one embodiment of the present invention provides a method for manufacturing multiple teeth from one isolated tooth germ comprising the epithelial and mesenchymal tissue layers, characterized in that it comprises the steps of:
(a) completely or partially splitting (dividing) one isolated tooth germ comprising the epithelial and mesenchymal tissue layers,
   wherein
   said splitting is characterized in splitting so that each splitted tooth germ portion respectively comprises a portion of said epithelial tissue layer and a portion of said mesenchymal tissue layer, and
(b) culturing said splitted tooth germs in vitro or culturing in vivo in a living animal other than humans to form multiple teeth.

Moreover, one embodiment of the method according to the present invention is characterized in that said step (a) is a step of partially splitting said "one isolated tooth germ comprising the epithelial and mesenchymal tissue layers" by ligating said "one isolated tooth germ comprising the epithelial and mesenchymal tissue layers" with a ligature.

Moreover, one embodiment of the method according to the present invention is characterized in that said "one isolated tooth germ comprising the epithelial and mesenchymal tissue layers" is in the developmental stage between the cap stage and the late bell stage.

Moreover, one embodiment of the method according to the present invention is characterized in that said step (a) is a step of completely splitting said "one isolated tooth germ comprising the epithelial and mesenchymal tissue layers" by cleaving said "one isolated tooth germ comprising the epithelial and mesenchymal tissue layers."

Moreover, one embodiment of the method according to the present invention is characterized in that said "one isolated tooth germ comprising the epithelial and mesenchymal tissue layers" is in the developmental stage between the cap stage and the early bell stage.

Moreover, one embodiment of the method according to the present invention is characterized in that in said step (a), said splitting is splitting so that each splitted tooth germ portion is uniform in size.

Moreover, one embodiment of the method according to the present invention is characterized in that said "one isolated tooth germ comprising the epithelial and mesenchymal tissue layers" is a tooth germ collected from a living animal.

Moreover, one embodiment of the method according to the present invention is characterized in that said step (b) is a step of arranging said each splitted tooth germ inside a device that can control the growth size of the tooth and culturing in vitro or culturing in vivo in an animal other than humans to form multiple teeth, wherein said device is in a configuration that can prevent said tooth germ from elongating to more than the maximum acceptable value, as well as in a configuration such that the tooth germs that are arranged inside can communicate with the exterior of said device.

Moreover, one embodiment of the method according to the present invention is characterized in that said step (b) is a step of arranging said each splitted tooth germ inside said device and culturing in vivo in an animal other than humans to form multiple teeth, wherein said device is further in a configuration that can prevent excessive pressure from tissues in said living animal being applied to said tooth germ.

Another embodiment of the present invention is characterized in that it is a tooth manufactured by a method comprising the following steps of:
(a) completely or partially splitting one isolated tooth germ comprising the epithelial and mesenchymal tissue layers,
   wherein
   said splitting is characterized in splitting so that each splitted tooth germ portion respectively comprises a portion of said epithelial tissue layer and a portion of said mesenchymal tissue layer, and
(b) culturing said splitted tooth germs in vitro or culturing in vivo in a living animal other than humans to form multiple teeth.

Yet another embodiment of the present invention is a method for repairing a lost tooth of an animal characterized in that it comprises transplanting a tooth manufactured by a method comprising the following steps to said site of loss:
(a) completely or partially splitting one isolated tooth germ comprising the epithelial and mesenchymal tissue layers,
   wherein
   said splitting is characterized in splitting so that each splitted tooth germ portion respectively comprises a portion of said epithelial tissue layer and a portion of said mesenchymal tissue layer, and
(b) culturing said splitted tooth germs in vitro or culturing in vivo in a living animal other than humans to form multiple teeth.

One embodiment of the method for repairing a lost tooth of an animal accroding to the present invention is characterized in that said animal is an animal other than humans.

It will be recognized by those skilled in the art that an invention of any combination of one or more characteristics of the present invention described above is also within the scope of the present invention.

### Effects of the Invention

According to the method for manufacturing a tooth according to the present invention, multiple teeth can be manufactured from one isolated tooth germ, thereby allowing to increase the absolute number of transplantatable tooth germs.

### Brief Description of the Drawings

Figure 1 shows a schematic diagram of splitting a tooth germ by ligation, as well as tooth germ tissue images at Day 2 and Day 6 from ligation.
Figure 2 shows tooth germ tissue images at Day 1 to Day 6 from ligation when the tooth germ was ligated at two places.
Figure 3 shows a schematic diagram of splitting a tooth germ by cleaving, as well as tooth germ tissue images at Day 4 and Day 6 from cleaving.
Figure 4 shows micro CT images and tissue images after 28 days from transplantation of a ligated tooth germ that was subjected to 4 days of organ culture and transplanted to the subrenal capsule of a mouse.
Figure 5 shows micro CT images and tissue images after 28 days from transplantation of a cleaved tooth germ that was subjected to 6 days of organ culture and transplanted to the subrenal capsule of a mouse.
Figure 6 shows tooth germ tissue images of tooth germs from fetal age 13 to 17 days which were each splitted and subjected to 6 days of organ culture.
Figure 7 shows micro CT images of a ligated tooth germ that were transplanted into the oral cavity of a mouse at Day 50 after transplantation.
Figure 8 shows the comparison of Shh and FGF4 gene expressions in ligated tooth germs at organ culture Day 1 to Day 6.
Figure 9 is a figure showing that bone remodeling via the periodontal ligament occurs in a tooth manufactured by one embodiment of the method of the present invention.
Figure 10 is a figure showing that nerves have penetrated into the periodontal ligament and the dental pulp of a tooth manufactured by one embodiment of the method of the present invention.
Figure 11 is a figure showing that nerves that have penetrated the tooth manufactured by one embodiment of the method of the present invention allow transmission of stimuli to the nerve center.

### Description of Embodiments

A "tooth" as used herein refers to a tissue comprising continuous layers of dentin on the inside and enamel on the outside, and means a tissue with orientation having tooth crown and tooth root, although it is not limiting and is a concept comprising also the periodontium such as the alveolar bone or the periodontal ligament. The tooth orientation can be identified by the arrangement of the tooth crown and the tooth root. The tooth crown and the tooth root can be visually confirmed based on the form or tissue staining and the like. The tooth crown refers to the portion having a layered structure of enamel and dentin, and the enamel layer does not exist in the tooth root.

The dentin and the enamel can be easily morphologically identified by those skilled in the art by e.g. tissue staining. Moreover, enamel can be identified by the presence of ameloblasts, and the presence of ameloblasts can be confirmed by the presence or absence of amelogenin. On the other hand, dentin can be identified by the presence of odontoblasts, and the presence of odontoblasts can be confirmed by the presence or absence of dentin sialoprotein. The confirmation of amelogenin and dentin sialoprotein can be easily carried out by a method known in the art, examples of which include in situ hybridization and antibody staining.

A "tooth germ" as used herein refers to an early embryo of a tooth that is destined to be a tooth in the future, and refers to those in the bud stage, the cap stage, and the bell stage commonly employed in tooth developmental stage, in particular a tissue where accumulation of dentin and enamel which is a characteristic of hard tissue of tooth is not recognized.

The tooth germ is composed mainly of the epithelial and mesenchymal tissue layers, and as the developmental stage progresses, the epithelial tissue layer may ultimately differentiate into the tooth enamel, and the mesenchymal tissue layer may ultimately differentiate into the tooth dentin, the dental pulp, the cementum, the periodontal ligament, and the alveolar bone.

"One isolated tooth germ comprising the epithelial and mesenchymal tissue layers" as used herein means that one isolated tooth germ employed in the present invention is composed substantially of the epithelial and mesenchymal tissues. Those skilled in the art will recognize that a tooth germ that comprises tissues other than the epithelial and mesenchymal tissues is also within the scope of the present invention.

A tooth germ in the "cap" developmental stage as used herein means a tooth germ at fetal age 14 to 15 days for mice. Moreover, for human deciduous teeth, a tooth germ in the "cap" developmental stage means a tooth germ at fetal age 7 weeks for the maxillary deciduous central incisor, the mandibular deciduous central incisor, the maxillary deciduous lateral incisor, and the mandibular deciduous lateral incisor, a tooth germ at fetal age 7.5 weeks for the maxillary deciduous cuspid and the mandibular deciduous cuspid, a tooth germ at fetal age 8 weeks for the maxillary first deciduous molar and the mandibular deciduous molar, and a tooth germ at fetal age 10 weeks for the maxillary second deciduous molar and the mandibular second deciduous molar. Further, for human permanent teeth, a tooth germ in the "cap" developmental stage means a tooth germ at fetal age 5 to 5.5 months for the maxillary central incisor, the mandibular central incisor, the maxillary lateral incisor, and the mandibular lateral incisor, a tooth germ at fetal age 5.5 to 6 months for the maxillary cuspid and the mandibular cuspid, a tooth germ at birth for the maxillary first premolar and the mandibular first premolar, a tooth germ at 7.5 to 8 months after birth for the maxillary second premolar and the mandibular second premolar, a tooth germ at fetal age 3.5 to 4 months for the maxillary first molar and the mandibular first molar, a tooth germ at 8.5 to 9 months after birth for the maxillary second molar and the mandibular second molar, and a tooth germ at 3.5 to 4 years after birth for the maxillary third molar and the mandibular third molar.

A tooth germ in the "bell" developmental stage as used herein means a tooth germ at fetal age 16 to 17 days for mice. Moreover, for human deciduous teeth, a tooth germ in the "bell" developmental stage means a tooth germ at fetal age 4 to 4.5 months for the maxillary deciduous central incisor and the mandibular deciduous central incisor, a tooth germ at fetal age 4.5 months for the maxillary deciduous lateral incisor and the mandibular deciduous lateral incisor, a tooth germ at fetal age 5 months for the maxillary deciduous cuspid, the mandibular deciduous cuspid, the maxillary first deciduous molar, and the mandibular deciduous molar, and a tooth germ at fetal age 6 months for the maxillary second deciduous molar and the mandibular second deciduous molar. Further, for human permanent teeth, a tooth germ in the "bell" developmental stage means a tooth germ at fetal age 3 to 4 months for the maxillary central incisor and the mandibular central incisor, a tooth germ at 10 to 12 months after birth for the maxillary lateral incisor, a tooth germ at 3 to 4 months after birth for the mandibular lateral incisor, a tooth germ at 4 to 5 months after birth for the maxillary cuspid and the mandibular cuspid, a tooth germ at 1.5 to 2 years after birth for the maxillary first premolar and the mandibular first premolar, a tooth germ at 2 to 2.5 years after birth for the maxillary second premolar and the mandibular second premolar, a tooth germ at birth for the maxillary first molar and the mandibular first molar, a tooth germ at 2.5 to 3 years after birth for the maxillary second molar and the mandibular second molar, and a tooth germ at 7 to 10 years after birth for the maxillary third molar and the mandibular third molar.

In the present invention, the type of tooth to collect the tooth germ from is not particularly limited, and collection of tooth germ can be from any tooth. In case of a human, however, the maxillary or mandibular third molar can be favorably employed in particular. The human maxillary and mandibular third molars are also called the wisdom teeth, and are the most late-developing teeth in the human oral cavity which develop into complete teeth at around 20 years old. The human maxillary and mandibular third molars often do not correctly erupt in the oral cavity and do not largely contribute to occlusion, and thus there are little disadvantages from their loss compared to other teeth.

In the present invention, the method for confirming the developmental stage of the tooth germ is not particularly limited, and confirmation can be made for example by visually or histologically observing the morphological change of the tooth germ epithelium accompanying development and differentiation. The developmental stage of the tooth germ can be confirmed because the morphology will be such that the epithelial tissue of the tooth germ will cover a portion of the mesenchymal tissue at cap stage, and the morphology will be such that the epithelial tissue will cover most of the mesenchymal tissue at bell stage. In addition to confirmation by visual or histological observation, the developmental stage of the tooth germ can also be confirmed by the formation of an enamel knot which is the signaling center that controls tooth development as an indicator. The method for confirming the formation of the enamel knot is not particularly limited, and well-known confirmation methods that can be employed by those skilled in the art can be employed, an example of which can include confirmation of the expression of Shh or FGF4 which are representative genes that are expressed in the enamel knot by in situ hybridization method. FGF4 is expressed in the 1st and 2nd enamel knots, and Shh is expressed at the interaction boundary surface between the epithelial and mesenchymal cells on the epithelial cell side during tooth germ development, as well as in the 1 st enamel knot.

The developmental stage of "one isolated tooth germ comprising the epithelial and mesenchymal tissue layers" that can be employed herein is not particularly limited as long as development will properly proceed after splitting. For example, a tooth germ in the developmental stage between the cap stage and the late bell stage is preferably employed when ligation with a ligature is employed as the method for splitting a tooth germ, and a tooth germ in the developmental stage between the cap stage and the early bell stage is preferably employed when cleaving is employed as the method for splitting a tooth germ.

The origin of the tooth germ employed herein is not particularly limited, and for example a tooth germ isolated from a living animal can be favorably employed. In the present invention, the animal to be the origin of the tooth germ is not particularly limited, and a tooth germ derived from any and all animals can be employed. For example, a tooth germ derived from a human, a pig, a cow, a monkey, a baboon, a dog, a cat, a rat, and a mouse can be employed.

In the present invention, the method for isolating a tooth germ from a living animal is not particularly limited, and a tooth germ can be isolated by those skilled in the art with an appropriate well-known method. For example, a tooth germ can be isolated from a tooth resected from an animal by separating only the tooth germ area with an injection needle.

In the present invention, the epithelial tissue refers to a tissue composed mainly of cells of the epithelial lineage, and the mesenchymal tissue refers to a tissue composed mainly of cells of the mesenchymal lineage. Confirmation that the tissue configuring the tooth germ is the epithelial and mesenchymal tissues can be made histologically (HE staining or immunostaining). For example, when the epithelial tissue is to be confirmed by immunostaining, confirmation can be made by immunostaining with an antibody against an epithelial tissue marker Shh.

"Splitting" a tooth germ as used herein is a concept that is not limited to e.g. completely separating a tooth germ into two or more tooth germ portions by ligation with a ligature or by cleaving, but also encompasses e.g. splitting a tooth germ into 2 or more tooth germ portions so that the tissue is partially connected by ligating it with a ligature without cleaving the tissue, or by partially cleaving it. It is also encompassed within the concept of the present invention to e.g. apply pressure with a plastic or metal clip so that the tooth germ tissue will not be crushed to split into individual tooth germ portions.

In the present invention, the aspects of splitting a tooth germ are not particularly limited, as long as it is splitted so that each splitted tooth germ portion respectively comprises a portion of the epithelial tissue layer of the tooth germ and a portion of the mesenchymal tissue layer of the tooth germ, and splitting may be of various aspects. When the tooth germ is splitted by ligation with a ligature, and when the tooth germ is splitted by cleaving, the division can be made e.g. in the plane that passes through the tooth germ midline (the line that passes though the central axis of the tooth germ in the epithelial-mesenchymal tissue direction, or the line that passes through the center of gravity of the epithelial tissue and the center of gravity of the mesenchymal tissue), or in the planes that are parallel to the plane that passes through the tooth germ midline. For example, the tooth germ may also be splitted into two or three individual tooth germ portions or more, but preferably the tooth germ can be splitted into two or three individual tooth germ portions.

Moreover, "splitting so that each splitted tooth germ portion is uniform in size" as used herein is not limited to the case where individual tooth germ portions are uniform in size in the strict sense, and it will be recognized by those skilled in the art that e.g. splitting so that each splitted tooth germ portion is uniform in size and respectively falls within a margin of error within approximately ±20%, more preferably approximately ±10% is also encompassed within the scope of the present invention. Moreover, the method for splitting so that each splitted tooth germ portion is uniform in size is not particularly limited, and for example, individual tooth germ portions can be splitted to be uniform in size by splitting so that individual tooth germ portions splitted by the method of the present invention respectively comprises a uniform amount of a portion of the epithelial tissue layer of the tooth germ and a uniform amount of a portion of the mesenchymal tissue layer of the tooth germ.

In one embodiment of the present invention, the tooth germ can be splitted by ligation with a ligature, but the method for ligating a tooth germ is not particularly limited. For example, a ligature can be applied to a tooth germ and ligated without cutting the tooth germ tissue. Preferably, for example, when ligating a mouse tooth germ, ligation can be performed so that the diameter of the ligature that surrounds the tooth germ is about 20 to 50 µm after ligation.

A "ligature" as used herein is not particularly limited as long as it can ligate a tooth germ tissue without crushing it. For example, a surgical suture (silk thread or nylon) and hair can be favorably employed as ligature. The thickness of the ligature is not limited as long as it can ligate a tooth germ without crushing it, and e.g. those having a thickness of 20 to 80 µm can be favorably employed.

In the present invention, the method for cleaving a tooth germ is not particularly limited, and various cleaving methods can be employed as long as it is a method that can cleave a tooth germ without crushing it. For example, a tooth germ can be cleaved by an injection needle, a surgical knife, or scissors.

In the present invention, the method for culturing the splitted tooth germ in vitro is not particularly limited, and those skilled in the art can appropriately select a well-known culture environment or medium, or a culture environment or medium pursuant thereto. For example, as the method for culturing the splitted tooth germ in vitro, the tooth germ can be cultured in an environment that is closer to that in vivo than an ordinary culture by culturing with a collagen gel and/or a cell culture insert. The collagen gel as used herein may be a collagen gel that is employed for organ or tissue culture, and the composition thereof is not particularly limited. For example, type I collagen gel, type III collagen gel, type IV collagen gel, and Matrigel can be employed as the collagen gel. By culturing the splitted tooth germ on a gel comprising collagen which widely exists in vivo, culturing can be carried out under conditions closer to that in vivo than culturing on glass or plastic. A cell culture insert as used herein is a permeable membrane employed for organ or tissue culture. By culturing cells or tissues on a cell culture insert, ingredients contained in the medium can be spread out to the top and bottom surfaces of the organ or tissue and culturing can be carried out under conditions closer to that in vivo.

"A device that can control the growth size of the tooth" as used herein when culturing a tooth germ inside a living animal means an appliance that is arranged to cover the tooth germ with the objective to prevent the tooth germ from elongating more than necessary. Such a device is sometimes referred to herein as a "spacer." Moreover, for example, when culturing in vivo such as the subrenal capsule, said device can play a role in preventing pressure from the renal capsule or excessive pressure from in vivo tissues being applied to the tooth germ. The shape of said device may be any shape as long as these objectives are achieved. For example, the shape of said device that can be favorably employed are cylindrical (ring-shaped) with open top and bottom or approximately cylindrical.

"A device that can control the growth size of the tooth" as used herein has a configuration that enables the tooth germs that are arranged inside to communicate with the exterior of said device. This is because since tooth germs are supplied nutrients etc. from tissues inside a living mammal or receive the effects of cytokine produced by animal cells, there is a need to secure a supply route for these substances to be delivered to tooth germs inside said device. In order to enable communication, the shape of said device can be such that it has pores formed thereon that allow substance supply, regardless of the size or the number. For example, it may have partial large pores such as a tubular shape, or have numerous small pores throughout said device such as a mesh. Moreover, it may not necessarily have pores if the material of said device enables permeation of substances.

In the present invention, the method for culturing the splitted tooth germ in vivo in an animal other than humans is not particularly limited. For example, the splitted tooth germ may be directly transplanted to an animal other than humans and cultured, or the splitted tooth germ may be placed in a spacer and transplanted to an animal other than humans together with the spacer and cultured.

In the present invention, the site for transplanting the splitted tooth germ to an animal other than humans for culturing is not particularly limited. For example, transplantation can be to the subrenal capsule, the subcutaneous, or the testis of an animal.

In the present invention, the method for transplanting the splitted tooth germ to an animal other than humans for culturing is not particularly limited. For example, when transplanting to the subrenal capsule of a mouse, an incision is made to the renal capsule, the renal capsule and the renal parenchymal are detached, and a splitted tooth germ (or a splitted tooth germ placed in a spacer) can be transplanted in between.

In the present invention, the animal that can be employed for transplantation for culturing the splitted tooth germ is not particularly limited as long as it is an animal other than humans, and any and all animal can be employed for transplantation. For example, pigs, cows, monkeys, baboons, dogs, cats, rats, and mice can be employed for transplantation.

In the present invention, the animal that can be employed for transplantation for culturing the splitted tooth germ is more preferably an immunodeficient animal other than humans. By employing an immunodeficient animal other than humans for transplantation, rejection by the body's immune function of can be prevented and the tooth germ can be efficiently cultured. An "immunodeficient animal" as used herein refers to an animal in which a portion or all of the body's immune function is deficient. The type of the deficient immune function is not particularly limited, but an animal that is deficient in immune function so that cells or tissues derived from other animal species transplanted to a living body are not rejected is preferred. For example, immunodeficient mice that can be employed are an SCID mouse, a nude mouse, an NOD mouse, an NOD-SCID mouse, an IL-2Rg knockout mouse, an RAG2 knockout mouse, an NOG mouse, and an RAG2/IL-2Rg double knockout mouse, preferably an SCID mouse. Moreover, for example, an SCID rat can be employed if it is an immunodeficient rat. Moreover, for example, an IL-2rg knockout pig can be employed if it is an immunodeficient pig.

In the present invention, the method for verifying the growth state of a tooth germ transplanted to an animal for culturing is not particularly limited. For example, the transplanted tooth germ may be directly analyzed without resection with a micro CT, or the transplanted tooth germ may be resected and histologically analyzed. When the growth state of the transplanted tooth germ is analyzed with a micro CT, the growth of the tooth from the transplanted tooth germ can be verified by e.g. verifying the formation of bone-like hard tissue around the tooth and the septum or the formation of the periodontal space. Moreover, when the growth state of the transplanted tooth germ is histologically (e.g. HE staining) analyzed, the growth of the tooth from the transplanted tooth germ can be verified by e.g. verifying the formation of the alveolar bone or the periodontal ligament.

In the present invention, the method for transplanting a tooth manufactured with the method of the present invention for tooth repair is not particularly limited, and multiple teeth manufactured with the method of the present invention may be directly transplanted, or multiple teeth manufactured with the method of the present invention may be individually separated before transplantation and each employed for transplantation.

In the present invention, the animal individual from which the tooth germ was isolated and the animal individual to which said tooth germ is transplanted after splitting and culturing may be different, but is preferably the same animal individual. For example, by splitting and culturing a tooth germ isolated from an animal individual and then employing it for transplantation to the same individual, immune rejection problems that may occur upon transplantation can be avoided, and the rate of establishment of the transplanted tissue can be improved.

The animal to be the subject of the method for repairing a lost tooth according to the present invention is not particularly limited. The method of the present invention can be employed for any and all animal tooth repair, but humans can be excluded from the subject when it is not preferred. For example, the method of the present invention can be employed for humans, pigs, cows, monkeys, baboons, dogs, cats, rats, and mice.

Note that the terms used herein are to be employed to describe particular embodiments and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meanings as those broadly recognized by those skilled in the art of the technology to which the present invention belongs. The terms used herein, unless explicitly defined otherwise, are to be construed as having meanings consistent with the meanings herein and in related technical fields, and shall not be construed as having idealized or excessively formal meanings.

Terms such as first and second are employed to express various elements, and it should be recognized that these elements are not to be limited by these terms. These terms are employed solely for the purpose of discriminating one element from another, and it is for example possible to describe a first element as a second element, and similarly to describe a second element as a first element, without departing from the scope of the present invention.

The present invention will now be more specifically described by Examples. However, the present invention can be embodied by various embodiments, shall not be construed as being limited to the Examples described herein.

### Examples

### (1. Laboratory Animals)

All animal experiments used in the Examples below were approved by the Laboratory Animals Ethical Committee at Tokyo University of Science, and experiments were conducted in compliance with the regulations thereof. SCID mice employed for animal experiments were purchased from CLEA Japan, Inc. (Tokyo, Japan) and bred in compliance with the animal experiment guidelines set by the National Institute of Health. In order to reduce the burden from experiments on the animals, operations were performed under general anesthesia by intraperitoneal injection of 5 mg/ml pentobarbital.

### (2. Establishing the method for splitting a tooth germ)

In order to investigate whether splitting of a tooth germ by ligation or cleaving is possible, the first molar tooth germ of a mouse at fetal age 14.5 days when the 1st enamel knot is formed was ligated or cleaved in the plane comprising the midline to split into individual tooth germ portions, and subjected to organ culture. In the present Example, a tooth germ splitted by ligation is referred to as a ligated tooth germ, and a tooth germ splitted by cleaving is referred to as a cleaved tooth germ. Moreover, in order to verify whether the tooth germ after organ culture is properly splitted, the ligated or cleaved tooth germ after organ culture was subjected to tissue analysis by HE staining.

### (2-1. Method for splitting a tooth germ)

The molar tooth germ was resected from the lower jaw of a fetal mouse at fetal age 14.5 days, and the first molar tooth germ area alone was separated with the tip of a 25 G needle. An 8-0 nylon surgical suture (Kakinuma Medical Inc.) was applied to the midportion of the tooth germ and ligated without cutting the tooth germ tissue. More specifically, a tooth germ ligated by applying a ligature on the plane comprising the midline of the subject tooth germ and loosely tying it (ligated tooth germ) was fabricated. The mesenchymal and epithelial lineage tissues in the tooth germ after ligation were both deformed, but both tissues maintained an uncleaved state immediately after ligation. Ligation was carried out so that the diameter of the ligature that surrounds the tooth germ after ligation is about 20 to 50 micrometers.

Moreover, as another method for splitting, a tooth germ ligated by applying ligatures to the subject tooth germ on planes that are parallel to the plane comprising the midline and split the tooth germ into three equal portions and loosely tying them (ligated tooth germ) was fabricated. A method similar to the method for applying a ligature on the plane comprising the midline was employed as the ligation method.

Further, as another method for splitting, a tooth germ cleaved by cleaving the plane comprising the midline of the subject tooth germ with a sharp 25 G needle so as not to crush the tissue (cleaved tooth germ) was fabricated.

### (2-2. Ex vivo organ culture method of ligated tooth germ)

In order to carry out ex vivo organ culture of the ligated or cleaved tooth germ fabricated by the above method for splitting a tooth germ, silicone grease was applied to a petri dish and Cellmatrix type 1-A (Nitta gelatin, Osaka, Japan) was added dropwise, the ligated or cleaved tooth germ was individually mounted, and left at 37°C for 5 minutes. After the collagen gel has solidified by leaving for 5 minutes, this was placed in a 12-well plate (BD) loaded with Cell Culture Insert (BD). To the plate were added 380 µl of 10% FBS (JBS), 0.1 mg/ml L-Ascorbic Acid (Sigma), and 2 mM L-Glutamine (GIBCO) supplemented D-MEM (Sigma), and cultured at 37°C under 5% CO₂ condition. The medium was exchanged every two days.

### (2-3. Tissue analysis by HE staining)

The ligated or cleaved tooth germ after organ culture was fixed with Mildform (Wako) for 24 hours. This was then embedded in paraffin (McCormick scientific), and 4 µm thick sections were fabricated from the ligated or cleaved tooth germ for histological analysis. The fabricated sections were subjected to hematoxylin-eosin staining (HE staining), And microscopic investigation was performed with Axio Imager A1 (Zeiss) installed with AxioCAM MRc5 (Zeiss).

### (2-4. Results)

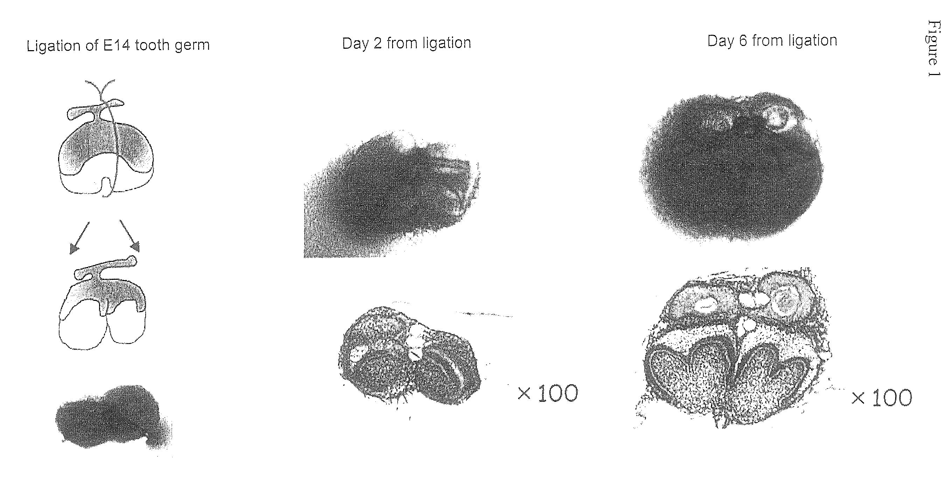

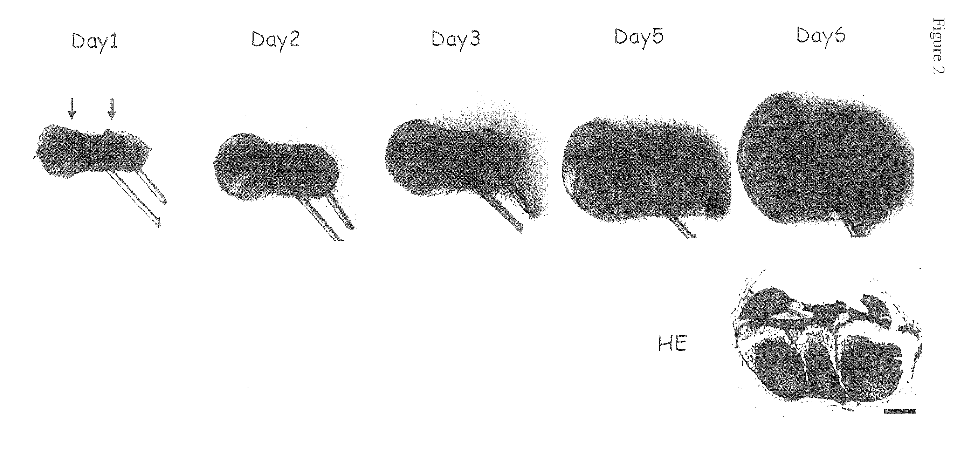

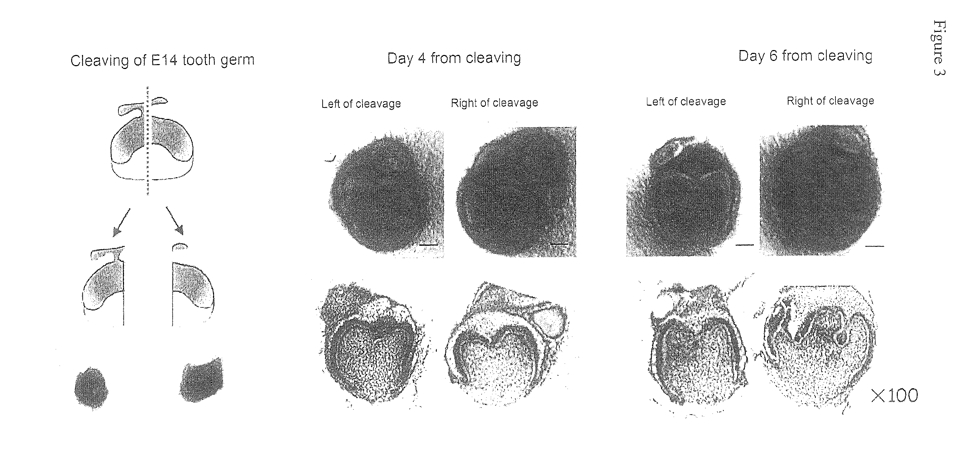

On Day 6 from start of organ culture, in the group where the tooth germ was splitted into two by ligation, the epithelial and mesenchymal tissues of the ligated tooth germ were severed and development of two normal tooth germs was confirmed (Figure 1). Moreover, similarly in the group where the tooth germ was splitted into three by ligation, development of three normal tooth germs was confirmed (Figure 2). This suggested that splitting a tooth germ by ligation is possible. Moreover, in the cleaved tooth germ, invagination of the epithelial and mesenchymal tissue was seen, thus suggesting that splitting a tooth germ by cleaving is similarly possible (Figure 3).

### (3. Heterotopic development of tooth germ)

The ligated or cleaved tooth germ was transplanted to the mouse subrenal capsule for observation of whether normal tooth germ is formed. More specifically, the ligated or cleaved tooth germ was transplanted to the mouse subrenal capsule, subsequently resected on Day 28, subjected to analysis by micro CT and histological analysis, and tooth germ formation of the ligated and cleaved tooth germs was evaluated.

### (3-1. Subrenal capsule transplantation of ligated tooth germ after ex vivo organ culture)

Under deep anesthesia, hair on the back of a C57BL/6 mouse where the kidney is located was shaved, and an incision of about 1 cm was made in the skin and the peritoneum. The kidney was then pulled out of the body from the incision site with ring tweezers (Feather). An incision of about 2 to 3 cm was made to the renal capsule with a razor (Feather), the ligated or cleaved tooth germ fabricated by a method similar to the above 2-1 was forced in between the kidney and the renal capsule. The kidney was then placed back into the body, and the muscle layer and the skin were each sutured. When transplanting the ligated tooth germ, the collagen gel attached around the ligated tooth germ was removed, this was placed into a spacer, and the ligated tooth germ was transplanted along with the spacer. After 28 days, it was evaluated whether the ligated or cleaved tooth germ has properly developed into a tooth.

### (3-2. Micro CT photography)

C57BL/6 mice which were transplanted the ligated or cleaved tooth germ were subjected to micro CT photography (In vivo Micro X-ray CT System; R_mCT, Rigaku Corporation) over a time course of immediately after transplantation and on Day 14, Day 28, Day 42, and Day 50 from transplantation day. The image data obtained by micro CT photography were evaluated over time with integrated image processing software (i-VIEW-3DX, Morita Corporation) for the binding between the ligated or cleaved tooth germ after transplantation and the recipient alveolar bone as well as for teeth development.

### (3-3. Tissue analysis by HE staining)

On Day 28 after transplantation, the ligated or cleaved tooth germ-derived teeth were resected from the recipient mice. The resected ligated or cleaved tooth germ-derived teeth were fixed with Mildform (Wako) for 24 hours. Moreover, the ligated or cleaved tooth germ-derived teeth after fixing by Mildform was subjected to two days of decalcification with 10% sodium citrate - 22.5% formic acid decalcification solution. This was then embedded in paraffin (McCormick scientific), and 6 µm thick sections were fabricated from the ligated or cleaved tooth germ-derived teeth for histological analysis. The fabricated sections were subjected to hematoxylin-eosin staining, and microscopic investigation was performed with Axio Imager A1 (Zeiss) installed with AxioCAM MRc5 (Zeiss).

### (3-4. Results)

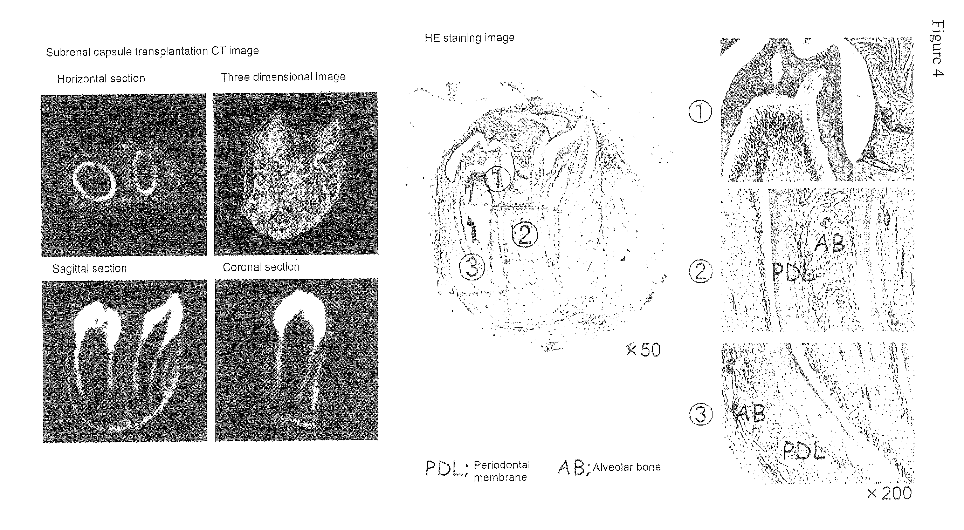

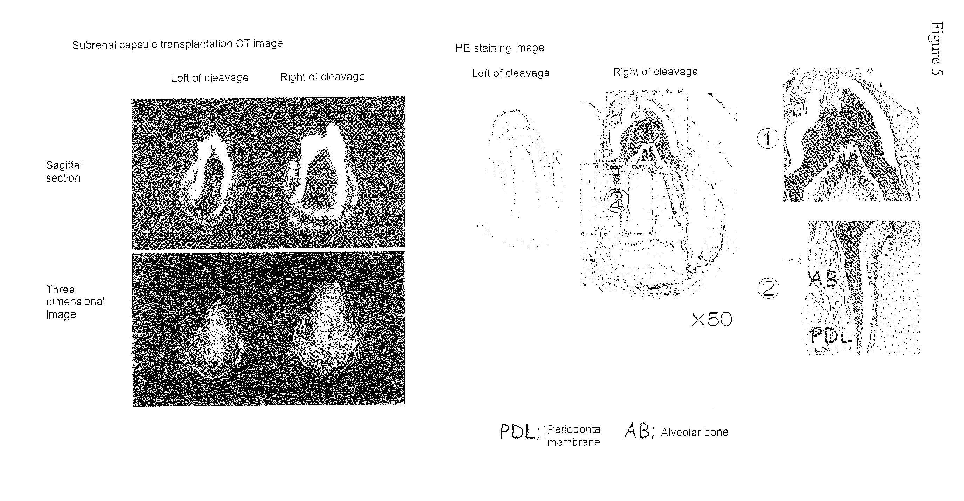

From the micro CT images, bone-like hard tissue is seen around the two teeth obtained from the ligated or cleaved tooth germ and the septum, and the periodontal space could also be confirmed (Figures 4 and 5). Further, it could also be confirmed from the tissue images after HE staining that the two teeth obtained from the ligated or cleaved tooth germ respectively had the alveolar bone and the periodontal ligament (Figures 4 and 5). From this, it was seen that the teeth obtained from the ligated or cleaved tooth germ has a tissue structure equivalent to a natural tooth, and it was shown that teeth had developed from splitted tooth germs.

### (4. Investigation of timing for splitting a tooth germ and method for splitting a tooth germ)

In order to further investigate the optimal timing for splitting a tooth germ as well as the method for splitting, tooth germs at each developmental stage from fetal age 13 to 17 days were ligated or cleaved, and the ligated and cleaved tooth germs obtained were subjected to 6 days of organ culture. Moreover, the ligated and cleaved tooth germs after organ culture were subjected to tissue analysis by HE staining. The method for splitting a tooth germ, the ex vivo organ culture method, and tissue analysis by HE staining were performed similarly to the methods described in the above 2-1, 2-2, and 2-3.

### (4-1. Results)

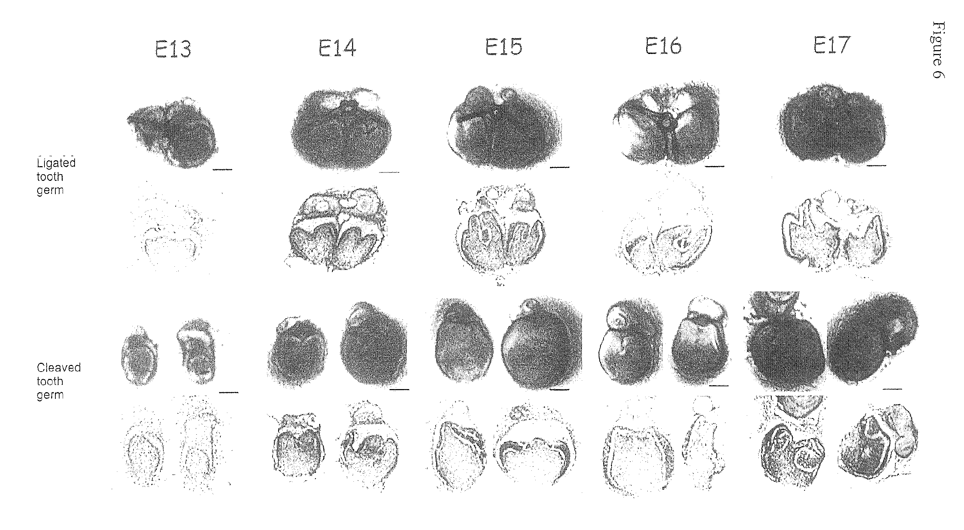

As a result, as shown in Figure 6, on organ culture Day 6, for tooth germs splitted from tooth germs derived from fetal age 13 days, there was a mixture of tooth germs where development do and do not proceed in both the ligated and cleaved tooth germs. For tooth germs splitted from tooth germs derived from fetal age 14, 15, and 16 days, the epithelial/mesenchymal tissue was completely splitted in both the ligated and cleaved tooth germs and development proceeded properly. However, for tooth germs splitted from tooth germs derived from fetal age 17 days, although the epithelial/mesenchymal tissue was completely severed in the ligated tooth germ, invagination of the epithelium was not seen, and some were seen with the mesenchymal tissue exposed in the cleaved tooth germ.

In mice, it is known that tooth germs at fetal age 14 to 15 days are in the cap stage and tooth germs at fetal age 16 to 17 days are in the bell stage. Accordingly, it was confirmed that a tooth germ properly develops regardless of whether it was splitted by ligation or cleaving, if the tooth germs were in the cap stage and early bell stage. It could also be confirmed that since the tooth germ splitted by cleaving may not be able to properly develop once it is in the late bell stage, the method for splitting a tooth germ in the late bell stage is preferably a method by ligation.

### (5. Analysis of tooth germ eruption in intraoral cavity transplantation model)

Analysis was performed with CT micro photography images to verify whether teeth development and teeth eruption having normal structure was possible by transplanting the ligated or cleaved tooth germ into the oral cavity.

### (5-1. Intraoral cavity transplantation of splitted tooth germ)

Under deep anesthesia, muscle fibers of the mandibular first molar alveolar socket of a 4 weeks-old C57BL/6 mouse were cut with a 25 G injection needle (Natume). Then, the tooth crown portion of the mandibular first molar was grasped with surgical tweezers (Natume) to extract the tooth. The mandibular first molar loss site was allowed to heal naturally in 2 to 3 weeks after the mandibular first molar tooth extraction. Subsequently, an incision of about 1 mm was made to the oral cavity mucosa of the mandibular first molar loss site with a surgical knife (Natume). After oral cavity mucosa incision, the alveolar bone was cut with a dental micromotor (Viva-Mate Plus) and a dental reamer (MANI) to form a transplantation fossa with a diameter of 0.8 mm and a depth of 1.2 mm.

As the tooth germ to be transplanted, a ligated tooth germ which was fabricated by a method similar to the above 2-1, and then subjected to 6 days of organ culture by a method similar to the above 2-2 was employed. The tooth germ after organ culture was employed for transplantation after removing the collagen gel attached to said tooth germ. Upon transplantation, the orientation of the tooth germ was verified and transplanted under a microscope so that the orientation of tooth eruption matches normal teeth. The transplantation site gingiva was sutured with an 8-0 nylon surgical suture (Bear Medic Corporation) after transplantation. Moreover, evaluation of whether the tooth germ properly develops was performed after transplantation until Day 50.

CT micro photography was carried out by a method similar to the above 3-2.

### (5-2. Results)

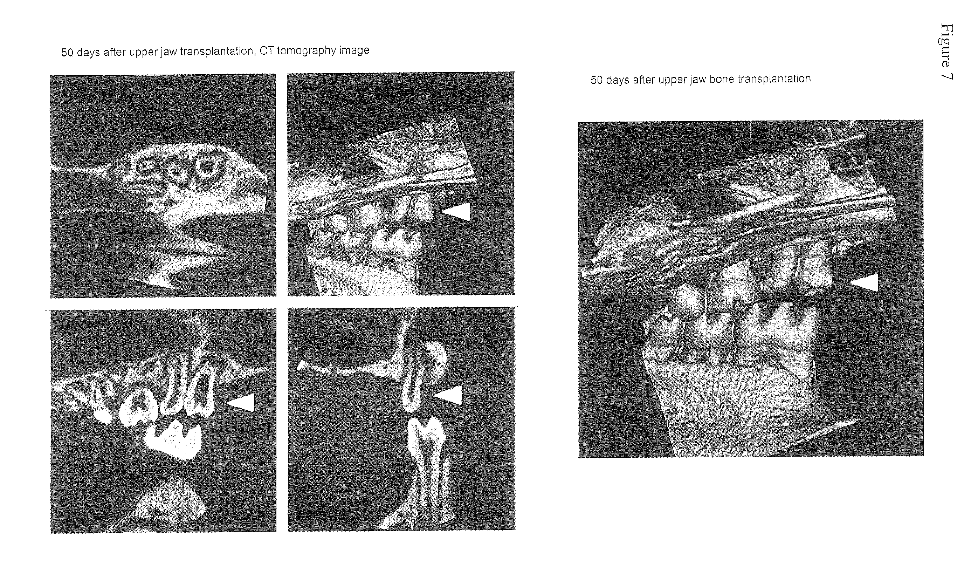

As a result, as shown in Figure 7, bone-like tissue was seen around the ligated tooth germ transplanted into the oral cavity, and the periodontal space was also seen. In other words, it was shown that multiple teeth can develop and erupt by transplanting tooth germs splitted from one tooth germ.

### (6. Analysis of development mechanism in splitting a tooth germ - in situ hybridization)

From the above results, development into teeth having normal structure was confirmed by transplanting the ligated or cleaved tooth germ to the mouse subrenal capsule or into the oral cavity or a mouse. From these results, gene analysis by in situ hybridization was carried out in addition to phenotypic analysis in order to compare gene expression in the developmental stage of a tooth germ splitted by ligation or cleaving with that in a natural tooth.

More specifically, in order to verify the formation of enamel knots in the developmental stage of tooth germ, expression analysis of Shh and FGF4 in a ligated tooth germ obtained by ligating the tooth germ at mouse fetal age 14.5 days (E14.5 ligated tooth germ) was carried out. Note that Shh and FGF4 are both representative genes expressed in the enamel knot which is the signaling center that controls tooth development. Shh is expressed at the interaction boundary surface between cells of the epithelial and mesenchymal lineage on the epithelial cell side during tooth germ development. Moreover, FGF4 is expressed in the 1st and 2nd enamel knots. The 2nd enamel knot expression site of FGF4 is the portion that will become the tooth cusp in the future. It is known that the 1 st enamel knot is formed in the tooth germ at mouse fetal age 14.5 days, and it is known that the 2nd enamel knot is formed in the tooth germ at fetal age 16 days.

Moreover, expression analysis of each gene was carried out with tooth germs from organ culture Day 1 to Day 6.

### (6-1. In situ hybridization)

Ligated tooth germs were fabricated from the tooth germ at mouse fetal age 14.5 days with a method similar to that described in the above 2-1, and subjected to organ culture by the method described in the above 2-2. Ligated tooth germs after organ culture was fabricated, and subjected to organ culture by the method described in the above 2-2. The duration of organ culture was set at 1 to 6 days as described above, and expression analysis of the genes in ligated tooth germs at organ culture Day 1, Day 2, Day 3, Day 4, Day 5, and Day 6 was carried out.

Each ligated tooth germ at organ culture Day 1 to Day 6 was fixed in 4% paraformaldehyde solution for 24 hours, and then subjected to 72 hours of decalcification with 10% formic acid sodium citrate - 22.5% formic acid decalcification solution. These were then immersed in 12.5% (w/v) and 25% (w/v) sucrose solutions for 12 hours each, and freeze embedded in OCT compound (Miles Inc, Naperville, IL). Ten µm thick sections (cryostat, CM3050S; Leica microsystems) were fabricated after embedding, and analysis of gene expression was carried out with Dig-labeled Shh probe or FGF4 probe.

### (6-2. Results)

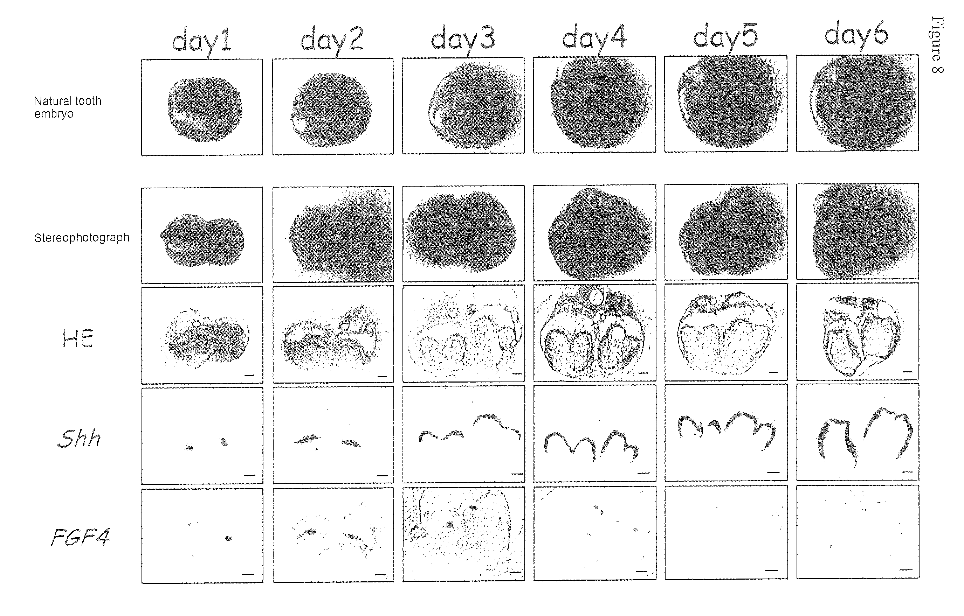

As a result, as shown in Figure 8, expression of FGF4 was seen in the ligated tooth germ from organ culture Day 1 to Day 4. Moreover, expression of Shh could be confirmed for the entire duration of up to organ culture Day 6. From the expression analysis result, the formation of the 1st enamel knot could be confirmed on organ culture Day 1. Moreover, the formation of the 2nd enamel knot could be confirmed on organ culture Day 3. Moreover, on organ culture Day 3 and after, the width of the tooth crown of the ligated tooth germ could be confirmed from the Shh region. In this way, the formation of a normal enamel knot could be confirmed even for ligated tooth germ subjected to organ culture.

### (7. Analysis of physiological functions of teeth developed from splitted tooth germs)

Analysis was carried out to determine not only whether the tooth germ-derived teeth which were splitted by the above method that developed and erupted in the intraoral cavity are histologically normal, but also whether they have physiological functions such as the function of the periodontal ligament or the function of connection with the nerve center equivalent to natural teeth. In order to determine whether migration of teeth due to physiological bone remodeling via the periodontal ligament occurs, an experiment was carried out by applying experimental corrective force to the developed teeth and evaluating by micro CT photography images and gene expression analysis. Specifically, after correction of the splitted tooth germ-derived teeth, migration of corrected teeth was verified by micro CT photography images. Moreover, in order to confirm that bone remodeling occurs in the bone surrounding the teeth which were applied corrective force, gene expression analysis of an osteoclast marker Csf-1 and an osteoblast marker OCN was performed.

Further, verification of whether nerves have penetrated inside the periodontal ligament and the dental pulp that were formed in the splitted tooth germ-derived teeth was made by immunohistological analysis. Moreover, analysis of whether transmission of stimuli from the peripheral to the central is possible was carried out by detection of c-fos protein expression in the medulla when performing the corrective experiment or the dental pulp exposure experiment.

### (7-1. Migration of teeth and bone remodeling due to correction)

For the experiment, a mouse to which a tooth germ was transplanted with a method similar to that described in the above 5-1 and eruption of two teeth derived from said tooth germ was confirmed was employed. In the present Example, among said two teeth, the one on the mesial side is referred to as the "mesial tooth" and the one on the distal side is referred to as the "distal tooth." The experiment was performed on a mouse under deep anesthesia. One enamel surface layer of the maxillary incisor was cut with a dental micromotor (Viva-Mate Plus), and the tooth surface layer was decalcified with a dental etching material. A Ni-Ti Closed Coil (TOMY INTERNATIONAL) that exerts a tractive force of 50 g in the range of 0.1 mm to 5.0 mm was installed between the splitted tooth germ-derived tooth and the maxillary incisor. One end of the coil was ligated to the cervix of the erupted mesial tooth with an 8-0 nylon surgical suture (Bear Medic Corporation), and further, the other end was ligated to the maxillary incisor cervix. The nylon suture ligated to the maxillary incisor cervix was fixed to the same with cured dental composite resin (GC) to prevent detachment of the device. The coil was removed after 6 days, fixed with a composite resin with the above two teeth secluded from each other, and analysis was carried out 7 days later by micro CT photography images with a method similar to that described in the above 3-2. Moreover, 10 µm thick sections were fabricated by a method similar to that described in the above 6-1, and gene expression analysis was carried out with Dig-labeled Csf-1 probe and OCN probe.

### (7-2. Results)

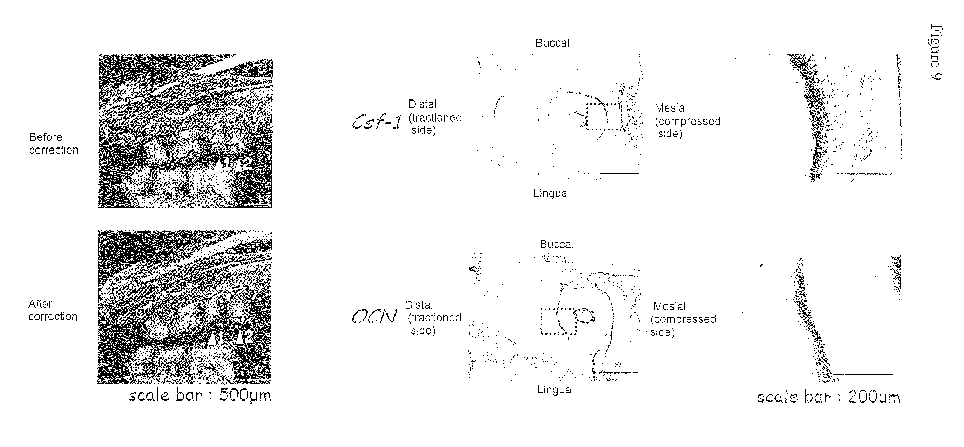

As a result, as shown in Figure 9, it was confirmed that the mesial tooth (Δ2) that was mesially tractioned (incisor side) migrated to the mesial side, and the distal tooth (Δ1) migrated to the distal side. Moreover, expression of an osteoclast marker Csf-1 was confirmed in the bone of the compressed side (mesial side) of the tractioned mesial tooth, and expression of an osteoblast marker OCN was confirmed in the bone of the tractioned side (distal side). From this expression analysis result, it became clear that bone remodeling via the periodontal ligament occurs also in the splitted tooth germ-derived teeth by applying corrective force.

### (7-3. Nerve penetration to tooth)

A splitted tooth germ was transplanted into the oral cavity of a mouse with a method similar to that described in the above 5-1, and 50 days later, the eruption of a splitted tooth germ-derived teeth was confirmed by micro CT photography. The upper jaw bone was then resected. The resected upper jaw bone was fixed with Mildform for 24 hours, and then subjected to 14 days of decalcification with 10% EDTA decalcification solution. This was then freeze embedded and 50 µm thick sections were fabricated. Immunohistological analysis was carried out with an anti-NF antibody (CHEMICON, rat anti-Neurofilament H, TA51) that stains nerve fibers for nerve fiber penetration analysis of said sections, an anti-NPY antibody (abcam, rabbit anti-Neuropeptide Y, poly) that stains the sympathetic nerve for penetration analysis of autonomic nerves, and an anti-CGRP antibody (AbD serotec, Goat anti-rat Calcitonin gene related peptide, poly) for penetration analysis of sensory nerves.

### (7-4. Results)

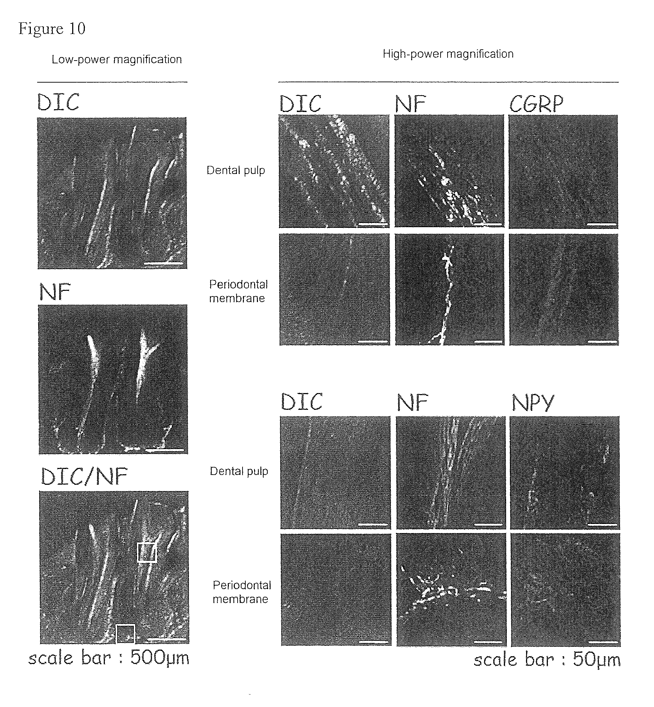

As a result, as shown in Figure 10, confirmation that nerves have penetrated inside the periodontal ligament and the dental pulp that were formed in the splitted tooth germ-derived teeth could be made. Accordingly, it became clear that splitted tooth germ-derived teeth have a tissue structure equivalent to natural teeth.

### (7-5. Reception of pain in nucleus tractus spinalis trigemini)

In order to analyze whether transmission of stimuli from the peripheral to the central is possible in the nerves of the splitted tooth germ-derived teeth confirmed in the above 7-3, 2 hours after the corrective experiment and the dental pulp exposure experiment, perfusion fixed and resected medulla was fixed with Mildform for 2 hours. After fixing, these were immersed in 12.5% (w/v) and 25% (w/v) sucrose solutions for 12 hours each, and freeze embedded in OCT compound (Miles Inc). After embedding, 50 µm thick sections were fabricated and immunohistological analysis was carried out with c-fos (Santa Cruz Biotechnology, Inc., c-Fos Antibody (4), poly) antibody.

The corrective experiment was carried out with a method similar to that described in the above 7-1. For the dental pulp exposure experiment, the tooth crown of the splitted tooth germ-derived teeth was cut with a dental micromotor and the dental pulp was exposed.

For perfusion fixation, first, under deep anesthesia, abdominal section was performed to the lower sternum so that organs could be observed, and an incision was made only to the skin in the pectoral region to expose the fascia. Next, the ensiform cartilage at the tip of the sternum was held with tweezers while the left and right ribs were dissected towards the shoulder, and the sternum was directly inverted towards the head. The diaphragm was dissected, the rib was excised to expose the heart, an injection needle was slowly inserted from the cardiac apex towards the left ventricle, and then the injection needle was fixed. An incision was made quickly to the swelled right atrium (right auricle), PBS (-) was flowed until exsanguination was complete, PBS (-) was switched to a paraformaldehyde solution once exsanguination was complete, and perfusion fixation was performed for approximately 20 minutes.

### (7-6. Results)

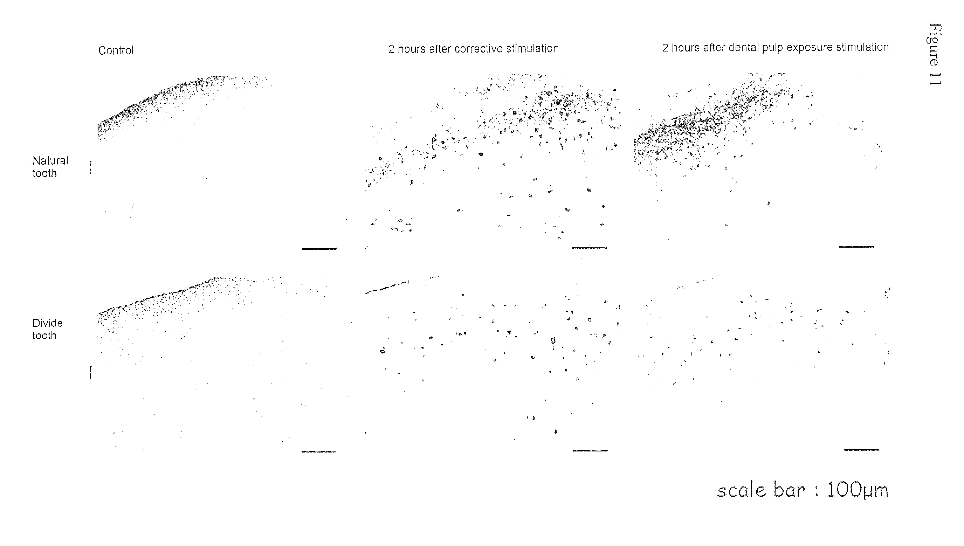

As a result, as shown in Figure 11, compared to the control without corrective stimulation or dental pulp exposure stimulation applied, dot-like expression of c-fos protein in the medulla was detected by stimulation due to corrective experiment and dental pulp exposure experiment in splitted tooth germ-derived teeth similarly to natural teeth. From this, it was shown that transmission of stimuli from the peripheral to the central is possible in splitted tooth germ-derived teeth similarly to natural teeth.

## Claims

1. A method for manufacturing multiple teeth from one isolated tooth germ comprising the epithelial and mesenchymal tissue layers, **characterized in that** it comprises the steps of:
(a) completely or partially splitting one isolated tooth germ comprising the epithelial and mesenchymal tissue layers,
wherein
said splitting is **characterized in** splitting so that each splitted tooth germ portion respectively comprises a portion of said epithelial tissue layer and a portion of said mesenchymal tissue layer, and
(b) culturing said splitted tooth germs in vitro or culturing in vivo in a living animal other than humans to form multiple teeth.

2. A method according to claim 1, **characterized in that** said step (a) is a step of partially splitting said "one isolated tooth germ comprising the epithelial and mesenchymal tissue layers" by ligating said "one isolated tooth germ comprising the epithelial and mesenchymal tissue layers" with a ligature.

3. A method according to claim 2, **characterized in that** said "one isolated tooth germ comprising the epithelial and mesenchymal tissue layers" is in the developmental stage between the cap stage and the late bell stage.

4. A method according to claim 1, **characterized in that** said step (a) is a step of completely splitting said "one isolated tooth germ comprising the epithelial and mesenchymal tissue layers" by cleaving said "one isolated tooth germ comprising the epithelial and mesenchymal tissue layers."

5. A method according to claim 4, **characterized in that** said "one isolated tooth germ comprising the epithelial and mesenchymal tissue layers" is in the developmental stage between the cap stage and the early bell stage.

6. A method according to any one of claims 1 to 5, **characterized in that** in said step (a), said splitting is splitting so that each splitted tooth germ portion is uniform in size.

7. A method according to any one of claims 1 to 5, **characterized in that** said "one isolated tooth germ comprising the epithelial and mesenchymal tissue layers" is a tooth germ collected from a living animal.

8. A method according to any one of claims 1 to 5, wherein said step (b) is a step of arranging said each splitted tooth germ inside a device that can control the growth size of the tooth and culturing in vitro or culturing in vivo in an animal other than humans to form multiple teeth,
wherein
said device is in a configuration that can prevent said tooth germ from elongating to more than the maximum acceptable value, as well as in a configuration such that the tooth germs that are arranged inside can communicate with the exterior of said device.

9. A method according to claim 8, **characterized in that** said step (b) is a step of arranging said each splitted tooth germ inside said device and culturing in vivo in an animal other than humans to form multiple teeth, wherein
said device is further in a configuration that can prevent excessive pressure from tissues in said living animal being applied to said tooth germ.

10. A tooth manufactured by a method according to any one of claims 1 to 9.

11. A method for repairing a lost tooth of an animal, comprising a step of transplanting a tooth manufactured by a method according to any one of claims 1 to 9 to said site of loss.

12. A method for repairing a lost tooth of an animal according to claim 11, **characterized in that** said animal is an animal other than humans.
